# EUROPEAN PATENT APPLICATION

(11) **EP 4 052 641 A1**
(43) Date of publication of application: **07.09.2022**
(21) Application number: 21160245.3
(22) Date of filing: 02.03.2021
(51) Int. Cl.: A61B 5/021, A61B 5/022, A61B 5/024, A61B 5/251, A61B 5/00, A61B 5/0205, H05K 1/02

(54) **VITAL-SIGN DETECTION DEVICE**

(71) Applicant: Murata Manufacturing Co., Ltd., Nagaokakyo-shi, Kyoto 617-8555 (JP)
(72) Inventor: Van Houwelingen, Marc, 2132 JC Hoofddorp (NL); Katsu, Hayato, Kyoto 617-8555 (JP); Nishida, Keisuke, Kyoto 617-8555 (JP); Nakamura, Yui, Kyoto 617-8555 (JP); Asai, Ryo, Kyoto 617-8555 (JP)
(74) Representative: Thoma, Michael

(57) **Abstract**

The present invention relates to a vital-sign detection device for detecting a vital sign, comprising a cuff on the inside of which a bladder at least partly made from a stretchable sheet material is provided to fit onto a body part, and at least one sensor placeable onto said limb by means of said cuff, wherein said at least one sensor includes a stretchable electronic element arranged on the stretchable sheet material of the bladder to be pressed onto the limb by deformation of said bladder.

## Description

The present invention relates to a vital-sign detection device for detecting a vital signs such as blood pressure, ECG, bio-impedance or oxygen saturation, comprising a cuff on the inside of which a bladder made at least partly from a stretchable sheet material is provided to fit onto a body part such as a finger or toe or another limb, and at least one sensor to be placed onto said body part by means of said cuff.

Monitoring of physiological parameters such as blood pressure, oxygen saturation, heart rate, respiratory rate, temperature or ECGs has become an important part of modern health care. Traditionally, such physiological parameters have been measured by rather bulky and expensive instruments at a hospital or a medical doctor's office. In order to allow for a comfortable monitoring at home and during travel, or over long periods of times such as repeatedly measuring blood pressure at intervals over an entire day, small-sized, easy wearable detection devices have been developed to allow for comfortable, but still precise monitoring of relevant vital signs.

Such portable detection devices may include ring- or sleeve-shaped attachments that can be slipped or put on someone's finger to monitor at least one of the aforementioned vital signs or physiological parameters at the finger. In a similar way, such attachments may be put onto a toe, whereas other attachments can be put onto a wrist, an ankle or another limb of the human body, wherein it is also possible to attach the device to a body portion or a limb of an animal body.

Depending on the vital-sign parameter to be detected and the technology for detecting such parameter, the vital-sign detector may include at least one sensor that needs contact to the skin or the body part's surface, whereas sometimes it may be allowed to position the sensing component onto the fabric of a shirt's sleeve or more generally, over the fabric of clothing or a plastic, hygienic layer of rubber gloves, for example, covering the skin. Irrespective of the type of contact, i.e. direct or indirect contact, it is usually helpful to press the sensor against the body part's surface to assure full contact and close fitting of the sensor to the body part. For example, an inflatable bladder may be used for pressing the sensor against the limb's surface, wherein such bladders or balloons are basically known from blood pressure measuring devices including a cuff that can be wrapped around a patient's arm and adjusted to a size matching the patient's arm, wherein the bladder in the cuff may be pumped up so the bladder expands and the inside of the cuff is pressed against the arm's surface.

A blood pressure monitoring device configured as a ring to be slipped onto a finger of a patient and having an inflatable bladder is known from document WO 2018/140207 A1, wherein such known detection device includes a rigid cuff in terms of a plastic or metal ring which may be adjusted in size to fit different finger sizes. In one ring sector of said cuff, a tactile sensor array is arranged on the inside of said ring-shaped cuff, wherein an opposite sector of the cuff is provided with an inflatable bladder arranged on the inside of the cuff so as to expand towards the center of the ring, thereby press-fitting the tactile sensor array on the opposite side to the finger surface.

Depending on the rotatory position of the cuff on the finger, however, the signals provided by the sensing components may be weak. In particular, when unexperienced users or dithering fingers attach the cuff, the signal quality may be bad or no signal at all may be provided, as the sensor array may not be appropriately allocated to the artery running through the finger.

Furthermore, prior art document US 2011/0251470 A1 discloses a device for peripheral impedance plethysmography which includes a stretchable electrode. Said stretchable electrode comprises an uninsulated stainless steel wire woven in the form of a tube that can be elongated, wherein the stretchable electrode is attached to a neoprene wrist band to be wrapped around an arm. Wrapping such neoprene wrist band around an arm or a leg of a human being works quite well, however, wrapping such wrist band around a finger is rather difficult due to the small size of a finger and the lack of space due to the neighboring fingers.

In view of the above, it is an object of the present invention to provide for an improved vital-sign detection device which avoids at least some of the aforementioned restrictions and disadvantages of the prior art and further develops the prior art in an advantageous manner. In particular, it is desired to provide for a vital-sign detection device which is easy to be mounted to a human body portion such as a limb, but nevertheless ensures reliable and sufficient contact to the limb's or body part's surface.

Furthermore, it is another objective of the present invention to provide for a vital-sign detection device allowing for cozy wearing and comfortable monitoring of the physiologic parameter even over longer terms.

A still further object may be precise detection of the relevant physiologic parameter even for unskilled, unexperienced users or elder people having dithering fingers.

A still further object is a miniaturized vital-sign detection device allowing precise monitoring of various vital parameters.

So as to achieve at least one of the aforementioned objectives, a vital-sign detection device is suggested which includes an electronic element fitted onto the body part by a bladder. More particularly, at least one sensor of the vital sign detection device includes an electronic element arranged on the stretchable sheet material of the bladder so as to be pressed onto the body part by deformation of said bladder.

Arranging the electronic element on the stretchable sheet material of the bladder allows for adaption of the electronic element to the body part contour and thus, full contact and close fitting of the electronic element to the body part surface. Furthermore, positioning of the electronic element is not restricted to a limited sector of the cuff left open by the bladder.

More particularly, due to positioning the electronic element on the bladder material, it becomes possible to choose different circumferential positions for the electronic element and/or more than one electronic element can be positioned at various positions since the electronic element position is no longer restricted to a ring sector not covered by the bladder as it was the case with the prior art. Due to such freedom in positioning the electronic element, it becomes easier to bring the electronic element into a position allowing the sensor to receive good signals from the body part to which the device is attached.

So as to make the cuff closely fit onto the respective body part, an interior space of the bladder may be filled with, for example, pressurized air or gas, or a pressurized fluid to pump up the bladder. When the interior space of the bladder expands due to the inflow of air, gas and/or fluid, the stretchable sheet material is deformed to snuggly fit onto the contour of the body part. At the same time, the electronic element gets into well-set or close fitting contact to the body part.

According to an advantageous aspect, at least a portion of said electronic element may be stretchable. More particularly, said electronic element may include a stretchable portion which is arranged on and/or in the stretchable material of the bladder, wherein it is also possible that the entire electronic element is stretchable.

When the electronic element itself is stretchable or deformable, it may compensate the deformation of the bladder and the changes in shape thereof.

Thus, the bladder may not only effect close fit of the cuff and the sensor onto the body part, but at the same time the bladder may be part of the electronic elements of the sensor and vice versa. More particularly, the electronic element may be sort of integrated into the bladder. Such electronic element may basically include various electronics such as a wiring or electrode which is usually formed on a printed circuit board and various electronic components or connections therebetween. For example, the electronic element may include photodiodes, LEDs, piezo elements, capacitors, microchips or other electronic components useful for sensing vital signs.

Generally, the electronic element may include at least one sensing component for sensing the parameter or vital sign of interest and providing a signal representing a characteristic thereof. Depending on the vital sign to be detected, different types of sensing components may be provided, wherein such different types of sensing components may be provided at the same time to allow the vital-sign detection device to detect different physiologic parameters and vital signs.

Said at least one sensing component may be stretchable or may at least include a stretchable portion which may be arranged on said stretchable material of the bladder. However, in the alternative, said sensing component does not have to be stretchable.

For example, the sensing component may include one or more photodiodes cooperating with one or more LEDs or more generally, one or more light sensors cooperating with one or more light sources so as to allow, for example, plethysmograms and/or the determination of blood pressure and/or the determination of oxygen saturation and hemoglobin levels. In addition or in the alternative, the sensing component may include other types of sensing elements such as one or more pressure sensing components and/or one or more temperature sensing components and/or one or more magnetic field generators cooperating with one or more magnetic field sensors and/or one or more piezo based transmitters and receivers.

Basically, the sensing component could be arranged directly on either side of the electronic element what allows easy connection in terms of short wirings and signal transmission. For example, an array of sensor elements may be provided onto the electronic element to measure the body part's impedance.

In addition or in the alternative, at least one sensing component may be positioned separate and/or spaced apart from the electronic element, wherein according to a further aspect of the present disclosure the sensing component may be positioned on the stretchable sheet material of the bladder with circuitry connecting the sensing component to the electronic element being provided on and/or in said stretchable sheet material of the bladder. Positioning the sensing component spaced apart from the electronic element on the stretchable sheet material of the bladder allows for choosing the appropriate position for the sensing component irrespective of the position of the electronic element. For example, a photo sensor component such as a photodiode cooperating with a light source such as an LED may be arranged on bladder portions substantially opposite to each other, whereas the electronic element may be positioned on another portion of the bladder which may be positioned in between the bladder portion supporting the sensing components.

According to a further aspect, the electronic element and the at least one sensing component may be positioned on opposite sides of the stretchable sheet material of the bladder. More particularly, the electronic element may be positioned on the exterior or skin contact side of the stretchable sheet material of the bladder (which may form the inner side of the ring-shaped device to be attached onto the finger), whereas the at least one sensing component may be positioned on the interior or radially outer side of the stretchable sheet material of the bladder (which may form the interior wall side facing the expandable volume chamber of the bladder). Such arrangement of the sensing component in the inner chamber of the bladder or on the interior side of the stretchable sheet material of the bladder shelters efficiently uses the available space, thereby rendering the device very compact in size.

So as to connect the sensing component on one side of the stretchable sheet material to the electronic element on the other side of the bladder's stretchable sheet material, the circuitry may go through this stretchable material from one side to the other side. For example, the connecting circuitry may include some vias going through the stretchable sheet so as to connect the sensing component to the electronic element on opposite sides thereof. The conductive material of such vias may be embedded in the stretchable sheet material of the bladder at least partially. More particularly, the vias may penetrate the stretchable sheet material in a sealed manner to allow pressurizing and inflating the bladder by means of pressurized air, pressurized gas and/or pressurized fluids and/or pressurized liquids.

In addition or in the alternative to circuitry going through the stretchable sheet material of the bladder from one side to the other side thereof, it is also possible to connect the sensing component arranged on one side of the sheet material to the electronic element positioned on the other side of the material by means of circuitry extending along opposite sides or opposite surfaces of the sheet material to a portion of the bladder where the sheet material overlaps and/or a portion of the stretchable sheet material projecting from the inflatable portion of the bladder.

More particularly, like the strap of a wrist watch, the stretchable sheet material of the bladder may form a loop with end portions of the strip-like sheet material overlapping each other. At such overlapping section, the interior side of one end portion of the sheet material may face the exterior side of the other end portion of the sheet material, thus allowing to contact circuitry on the interior side to circuitry on the exterior side. Thus, the sensing component on the interior side of the stretchable sheet material may be connected to the electronic element on the exterior side of the stretchable sheet material without penetrating or perforating said stretchable sheet material.

For example, electronic elements on an interior side of the stretchable sheet material may extend to one end portion thereof, whereas electronic elements on the opposite side of the stretchable sheet material may extend to the other end portion thereof so, when forming the sheet-like material into a loop-like configuration, the electronic elements may contact each other at the end portions overlapping each other.

In addition or in the alternative of connecting the circuits on the opposite sides to each other at such overlapping loop portion, the circuits also may extend to a common end portion extending beyond and/or projecting from the bladder portion or loop portion so the circuits may be connected at such common end portion outside the bladder where the stretchable material is not subject to expansion.

At an outer side of the bladder, the cuff may include an outer ring element made from a non-stretchable material, for example a fiber reinforced plastic material or fabric or made from a rigid plastic and/or metal material. Despite being non-stretchable, the material may flex or bend but not expand or contract as it is the case with a sheet of paper or a fiberglass strip. In the alternative, a rigid material like metal may form such outer ring element.

Such non-stretchable outer ring element may support the bladder at an outer peripheral side and/or against radial expansion and/or radial deflection so the bladder may apply the desired pressure onto the body part on which the ring-shaped device is positioned. The bladder may extend along the entire inner side of said outer ring element to form a 360° inner ring element. It is preferable since the pressure then presses the stretchable sheet material to the whole circumference of the body part and more accurate measurement or detection of the vital-sign can be achieved. However, this is not necessary, but the bladder may cover only a portion of the inner surface of the outer ring element and/or extend along less than 360° such as for example 270°.

Said outer ring element made from non-stretchable material may form a substantially flat ring having a substantially rectangular or flattened cross-section. The bladder may form a ring-shaped tube the outer side of which may contact the inner side of the outer ring.

However, in the alternative, the outer ring element made from non-stretchable material may have a substantially U-shaped cross-section and/or may have a concave inner ring side contour forming a sort of chute or channel extending along the inner side of the outer ring element. When using the aforementioned tube-like bladder, the outer side of the bladder may nestle into such concave inner ring side to give support not only in the radial direction but also in the axial direction to some extent.

However, according to a further aspect, the bladder does not need to have a tube-like configuration. More particularly, the stretchable sheet material of the bladder does not need to have a tube-like configuration with a ring-shaped cross-section, but the stretchable sheet material of the bladder may form an inner shell cooperating with the outer ring element made from non-stretchable material forming the outer shell. The stretchable sheet material forming the inner shell may be connected to or fixed to the outer ring element along side edges of the strip-like, stretchable sheet material.

More particularly, the outer ring element may have a substantially U-shaped cross-section or a concave, chute-like inner ring side with a pair of webs or flange-like rims extending towards the center of the ring-shaped device and/or substantially radially inwards. The stretchable sheet-material of the bladder may be fixed to said webs or rims of the non-stretchable outer ring element so the expandable volume of the bladder may be defined in part by the stretchable sheet material and in part by the non-stretchable outer ring element. Due to the inwardly extending side rims of the outer ring element, a chamber is formed even when the stretchable sheet material is not bulging or otherwise expanded or substantially flat, thereby giving space for accommodating the aforementioned sensing components such as LEDs, photodiodes, pressure sensing elements or other sensing components.

Furthermore, due to the inwardly extending side rims of the substantially rigid outer ring element, a dead space left between the stretchable sheet material and the outer ring body which facilitates uniform inflation of the bladder along the circumference of the body part and in particular over the full circumference of a finger when said bladder forms a closed 360° ring.

In the alternative, it also would be possible to attach the stretchable sheet material with some slack or some excess in material to a substantially flat outer ring element so the stretchable material, already in its non-expanded state, may form a substantially U-shaped cross-section to define a dead space between the outer ring element and the bulging stretchable sheet material.

So as to expand the bladder to achieve pressure onto the body part surface, the detection device may include a pump for pumping pressurized gas or air into the inner bladder chamber, wherein such pump may be positioned outside said inner bladder chamber and more particularly, on an outer side of the outer ring element made from non-stretchable material. For example, the pump may be arranged on the outer circumferential side of said outer ring element, wherein a pressurizing gas channel communicating the pump with the inner bladder chamber may extend through said outer ring element.

Said pump may include a micro-blower. Such micro-blower may supply sufficient air flow and pressure and achieves a very compact design. The micro-blower may be integrated into the outer ring element of the cuff.

The signals and/or values collected by the sensing component and/or the electronic element may be processed and/or analyzed in different ways and/or at different locations. For example, the portable device itself may include a signal processor and/or a data analysis unit so as to determine the desired vital sign and/or physiologic parameter from the sensing signals. The signal analysis unit may be integrated into the cuff device. Furthermore, a display unit may be provided at the wearable device to display the relevant information relating to the desired vital sign. For example, similar to traffic lights, there may be red, green and yellow lights in terms of LEDs for example, to indicate the determined vital signs are okay, not okay or still okay, but close to not okay. In addition or in the alternative, more sophisticated display units may be provided such as a display screen indicating precise values of the respective vital sign such as blood pressure values or oxygen saturation values. Other display units such as acoustic output may be provided.

In addition or in the alternative to such data analysis and/or information display at the cuff device itself, the signals collected by the sensing component and/or partially preprocessed signals may be transmitted to an external analysis and/or display system which may include, for example, a data server or a computing unit at a hospital or a doctor's office. So as to allow for such data and/or signal transmission, the detection device to be attached to a body part may include a data transmission component which may include a wired transmitter, a Bluetooth transmitter, a radio transmitter and/or other mobile data communication components. Such data transmission device may be incorporated into the ring-shaped cuff or, in the alternative or in addition, a data transmission component may be releasably connected to the cuff or another element of the detection device which may include a data interface.

Further details and optional embodiments of the invention will become apparent from the following description of examples of the invention and the drawings corresponding thereto. In the drawings show:
- Fig. 1:: a cross-sectional view of a vital-sign detection device according to a first embodiment including a ring-shaped cuff to be attached onto a finger of a human body,
- Fig. 2:: an illustration of the collection and analysis of the sensor signals to determine the vital sign of interest,
- Fig. 3:: a partial perspective and partial cross-sectional view of a vital-sign detection device according to another embodiment which includes a rigid outer ring with a U-shaped cross-section to which stretchable sheet material of the bladder is attached to jointly define the interior bladder chamber,
- Fig. 4:: a plane view and a side view of the stretchable sheet material of a bladder of the device having electronic elements on the inner side and on the outer side of the sheet material to be connected with each other at an overlapping end portions when forming the stretchable sheet material into a loop,
- Fig. 5:: a perspective view of the loop formed by the stretchable sheet material of Fig. 4, with the inside and outside electronic elements connected to each other at the overlapping end portions,
- Fig. 6:: plane and side views of a stretchable sheet material of a bladder provided with electronic elements on the exterior side and sensing components on the interior side of the bladder, wherein said electronic elements and sensing components are connected to electronic components at an extended end portion via circuitry extending along the interior and exterior sides, and
- Fig. 7:: a perspective view of the loop formed by the stretchable sheet material of Figure 6.

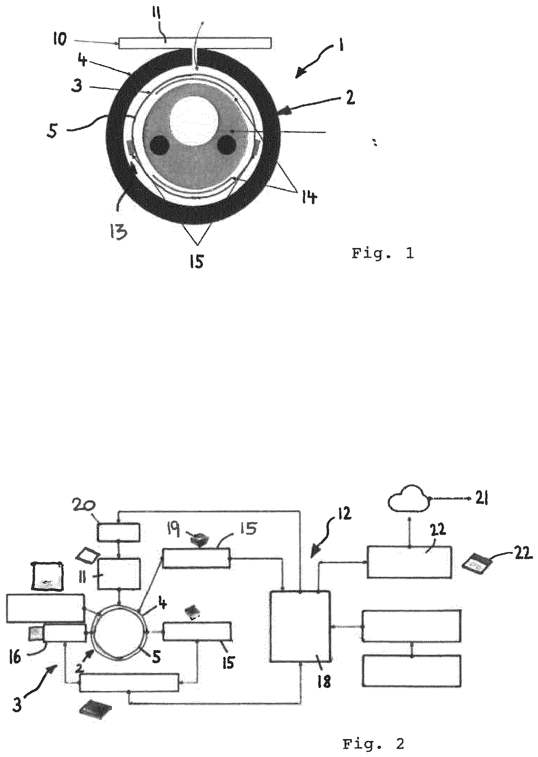

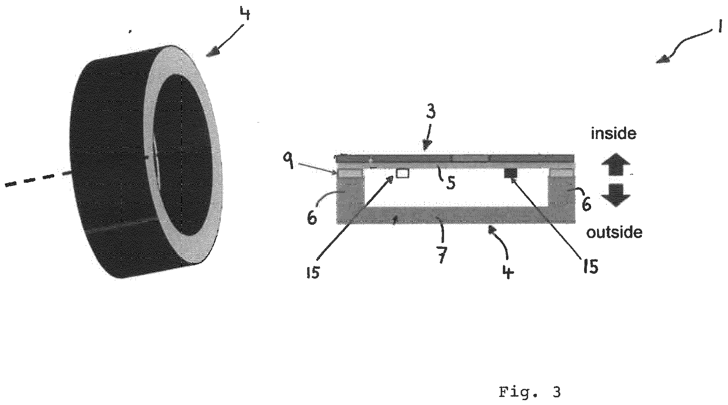

As can be seen from figure 1 and figure 3, the vital-sign detection device 1 includes a ring-shaped cuff 2 which may be configured to be put onto a finger of a human being. The size of the cuff 2 is adapted to snuggly fit onto the finger, at least when a bladder 3 arranged at the inner circumferential side of the cuff 2 is expanded or inflated.

The cuff including the bladder on the inside thereof, however, also may be configured to snuggly fit onto other limbs such as a toe, the wrist of an arm or the ankle of a leg or other body portions at which a vital sign can be measured. Due to the cuff having a deformable bladder on the inside, the detection device may be miniaturized to fit onto small body portions such as a small finger, and also allows an invasive use, wherein, for example, the cuff may be mounted around an artery.

The cuff 2 may include an outer ring element 4 which may be made from a non-stretchable material such as a fiber reinforced plastic material or a rigid plastic and/or metal material.

Along the inner circumferential side of said ring element 4, said bladder 3 is provided which itself may form a closed ring in terms of a 360° ring covering substantially the entire inner peripheral surface of the outer ring element 4. However, as mentioned before, said bladder 3 may extend along only a portion of said inner peripheral side and may have a C-shape or extend, for example, along about 180° to 270° or another portion of a circle.

The bladder 3 may have a tubular configuration with a radially outer side contacting the inner peripheral side of the outer ring element 4. Said tubular bladder 3 may define an interior inflation chamber or bladder chamber which may be filled with air or a gas or another fluid or liquid so as to expand the bladder 3.

The bladder 3 may be formed from a stretchable sheet material 5 which may form the aforementioned tube having a closed, substantially ring-shaped cross-section.

Advantageously, the stretchable sheet material may be an elastic material which is elastically deformable. For example, the stretchable sheet material may be rubber or a rubber-like plastic material.

As it is shown by figure 3, the bladder 3 may be defined partly by the outer ring element 4 and partly by the stretchable sheet material 5. More particularly, the outer ring element 4 may have a U-shaped cross-section with a pair of inwardly extending, flange-like rims 6 which are connected to each other by an outer crosswise extending portion 7 bridging the distance between said side rims 6.

Said outer crosswise extending portion 7 may form the outer peripheral side of the ring element 4.

On the other hand, the stretchable sheet material 5 bridges the distance between the rims 6 and forms the inner peripheral side of the cuff 2. Due to the U-shaped cross-section of the outer ring element 4, a dead space is defined between the stretchable sheet material 5 and the outer crosswise extending portion 7, said dead space forming the interior bladder chamber 8 into which pressurized air or gas may be supplied to inflate or expand the bladder 3, more particularly, the stretchable sheet material 5 may bow or bulge or belly inwardly towards the center of the ring when the interior chamber 8 of the bladder 3 is filled with pressurized gas, air or liquid.

As can be seen from figure 3, the sheet material 5 may be fixed and sealed to the free ends of the rims 6 or another portion of said rim 6. For example, the stretchable sheet material 5 may be glued to the rims 6, for example by means of an adhesive layer 9.

As can be seen from figure 3, the cuff 2 may have a configuration similar to a tubeless vehicle wheel, wherein different to such tubeless vehicle wheel, the tire forms the inner peripheral side and the rigid rim forms the outer peripheral side of the wheel.

So as to inflate and expand the bladder 3, a pump 10 may be provided which may be a micro blower. Said pump 10 may be arranged at and/or attached to the outer peripheral side of the cuff 2, wherein a communication channel may extend through the outer ring element 4 to fill air or gas into the interior bladder chamber 8, cf. figure 1. It is also allowed for a pump not being directly attached to the cuff but being connected with a tube to the ring device.

The pump 10 may be controlled by a control unit 12 which may include electronics such as a processing unit and a storage, wherein such control unit 12 may be part of the detection device 1.

So as to detect relevant vital signs such as blood pressure, oxygen saturation or ECG, at least one sensor 13 may be provided so as to placed onto the finger or another body part of the human body by means of said cuff 2.

More particularly, said sensor 13 may include at least one electronic element 14 which is positioned on said stretchable sheet material 5 of the bladder 3. More particularly, the electronic element 14 may be positioned on and/or fixed to the inner peripheral side of the bladder 3 which is formed by the exterior surface of the stretchable sheet material 5, i.e. the surface of the sheet material 5 facing away from the interior chamber 8 and towards the center of the ring. Due to its position on the inner side of the stretchable sheet material 5, the electronic element 14 achieved good electric contact to the anatomical surface of the body part. The electronic element 14 may fully contact the surface of the body part and snuggly fit onto the contour thereof.

The electronic element 14 may include a tactile sensor array formed from, for example, a plurality of individual capacitive sensors, and/or an array of different type of sensors forming such sensor array. More generally, the electronic element 14 may include integrated circuits which are usually formed in a printed circuit board, electronic components or other elements, wherein said electronic element 14 may be part of the bladder 3 in terms of taking part in deformations of the bladder and compensating changes in shapes thereof.

Irrespective of the sensor function of the electronic element 14, the sensor 13 may further include sensing components 15 positioned spaced-apart and/or separate from the electronic element 14. Such sensing components 15 may include a photodiode and a light source such as an LED 16 cooperating therewith, wherein other sensing components such as temperature detectors or pressure detectors may be provided.

As can be seen from figures 1 and 3, such sensing components 15 may be positioned on the stretchable sheet material 5 of the bladder 3, wherein the sensing components 15 may be positioned on a side of the stretchable sheet material 5 opposite to the electronic element 14. More particularly, the sensing components 15 may be positioned on the outer surface, i.e. the interior surface of the stretchable sheet material 5 facing the expandable interior bladder chamber 8. In other words, the sensing components 15 may be accommodated within the interior bladder chamber 8.

To allow the sensing components 15 visual contact to the body part, the stretchable sheet material 5 may be transparent for the transmitter and sensing elements. In addition or in the alternative, the stretchable sheet material 5 may include a transparent window portion through which the transmitting and sensing components 15 may look onto the body part.

So as to electrically connect the sensing components 15 to the electronic element 14 and/or to allow signal transmission between the sensing components 15 and said electronic element 14, circuitry may be provided on and/or in said stretchable sheet material 5. For example, the circuitry may include vias penetrating the sheet material 5 and connecting the sensing components 15 on the one side of the sheet material 5 to the electronic element 14 on the other side thereof.

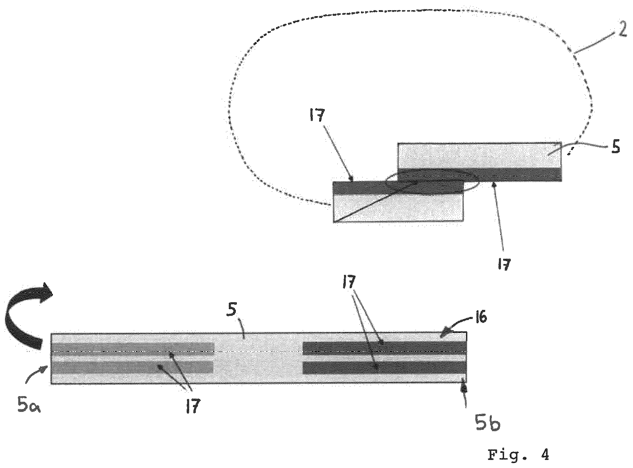

In addition or in the alternative to such vias penetrating the sheet material 5, the circuitry may include conductive circuits 17 and/or electronic elements and/or transmitting elements extending along the outer side of the stretchable sheet material on the one hand and along the inner side thereof on the other hand, wherein such circuit 17 may extend to opposite end portions 5a and 5b of the stretchable sheet material 5, as can be seen from figure 4.

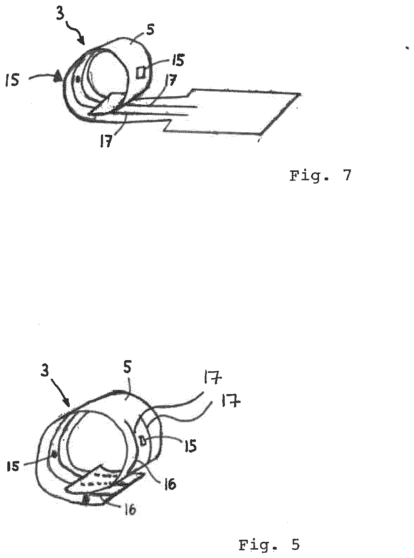

When forming a loop with said stretchable sheet material 5 so that said end portions 5a and 5b overlap each other, cf. figure 5, the circuits and/or electronic elements 17 on the outer side of the stretchable sheet material 5 may contact the circuits or electronic elements 17 on the inner side of said stretchable sheet material 5. Thus, the electronic element 14 and the sensing components 15 may be positioned on opposite sides of the stretchable sheet material 5 and nevertheless may be connected to each other without perforating the sheet material. Such configuration helps in keeping the bladder chamber 18 sealed even under higher pressures.

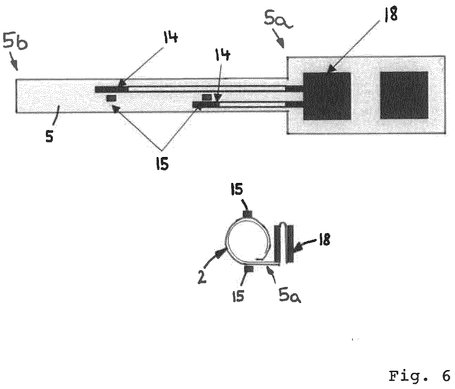

Another example of connecting electronic components on one side of the stretchable sheet material 5 and the electronic element 14 on the other side of the stretchable sheet material 5 without perforating the stretchable sheet material 5 is shown by figures 6 and 7, wherein circuits 17 from the sensing components or electronic elements may extend along the outer side and along the inner side of the stretchable sheet material 5, wherein such circuits 17 may run from the sensing component 15 or the electronic element 14, respectively, to a common end portion 5a of the stretchable sheet material 5 where the circuits 17 may be connected to electronic components such as a microchip, a pump driver, data analysis chips or data transmission elements. In addition or in the alternative, the common end portion 5a may be provided with contact elements at which the circuits 17 may terminate, wherein said contact elements 18 may be connected to each other and/or to further electronics elements, as it is shown by the side view of figure 6.

As can be seen from figure 2, the electronics of the detection device 1 may include a processor 18 which communicates with, for example, a pressure sensor 19 measuring the pressure inside the chamber 8 of the bladder 3 and/or the pressure of the bladder 3 against the body part, and/or with other sensing components 15 such as a one or more photodiodes and one or more light sources such as LEDs cooperating therewith to generate plethysmograms.

Furthermore, the processor 18 may communicate with a driver 20 for driving and operating the pump 10 to expand the bladder 3.

Said processor 18 may analyze the signals of the sensing component to determine the desired physiologic parameters or vital signs and/or may transmit the signals and/or the analyzed information to other data processing units 21 such as servers and computers at a hospital, a doctor's office or a home. As can be seen from figure 2, the detection device 1 may be provided with a data communication component 22 which may operate wireless and/or which may include a network transmission component.

As can be further seen from figure 2, other auxiliary equipment such as charging and/or power management systems, for example, a battery or another energy supply unit may be connected to the detection device 1.

## Claims

1. Vital-sign detection device for detecting a vital sign, comprising a cuff (2) on the inside of which a bladder (3) at least partly made from a stretchable sheet material (5) is provided to fit onto a body part, and at least one sensor (13) placeable onto said body part by means of said cuff (2), **characterized in that** said at least one sensor (13) includes an electronic element (14) arranged in and/or on the stretchable sheet material (5) of the bladder (3) to be pressed onto the body part by deformation of said bladder (3).

2. Vital-sign detection device according to the preceding claim, wherein at least a portion of said electronic element (14) is stretchable and/or wherein said electronic element includes a stretchable portion arranged in and/or on the stretchable sheet material (5) of the bladder (3).

3. Vital-sign detection device according to anyone of the preceding claims, wherein said sensor (13) includes at least one sensing component (16) arranged on said stretchable sheet material (5) and connected to said electronic element (14).

4. Vital-sign detection device according to the preceding claim, wherein said at least one sensing component (15) and said electronic element (14) are positioned on opposite sides of said stretchable sheet material (5).

5. Vital-sign detection device according to claim 3 or 4, wherein said at least one sensing component (15) is accommodated in an interior bladder chamber (8), whereas said electronic element (14) is positioned on an exterior side of the stretchable sheet material (5) facing the limb.

6. Vital-sign detection device according to anyone of claims 3 to 5, wherein circuitry connecting the sensing component (15) to the electronic element (14) includes vias extending through the stretchable sheet material (5) from one side to the other side thereof.

7. Vital-sign detection device according to claims 4 or 5, wherein the electronic element (14) is connected to the sensing component (15) without perforating the stretchable sheet material (5) by means of circuitry including circuits (17) extending along inner and outer sides of the stretchable sheet material (5) to opposite end portions (5a, 5b) thereof wherein said end portions (5a, 5b) overlap each other and form together an overlapping loop portion at which the circuits (17) on the inside and outside of the stretchable sheet material (5) contact each other.

8. Vital-sign detection device according to claims 4 or 5, wherein the electronic element (14) is connected to the sensing component (15) without perforating the stretchable sheet material (5) by means of circuitry including circuits (17) extending along inner and outer sides of the stretchable sheet material (5) to an end portion (5a) thereof wherein said end portion (5a) extends beyond and/or projects from an inflatable bladder portion, in particular from a loop formed by said stretchable sheet material.

9. Vital-sign detection device according to one of the preceding claims, wherein the cuff (2) includes an outer ring element (4) made from a non-stretchable material, wherein the bladder (3) extends along an inner peripheral side of said outer ring element (4) and is at least radially supported by said outer ring element (4).

10. Vital-sign detection device according to the preceding claim, wherein said outer ring element (4) has a U-shaped cross-section and/or a concave inner peripheral ring surface with a pair of inwardly extending rims (6) to which the stretchable sheet material (5) is attached so that an interior bladder chamber (8) is defined partly by said stretchable sheet material (5) and partly by said outer ring element (4).

11. Vital-sign detection device according to anyone of the preceding claims, wherein a pump (10) is provided for inflating said bladder (3), wherein preferably said pump (10) is arranged at an outer peripheral side of said cuff (2) and communicates with the interior bladder chamber (8) via a channel going through an outer ring portion of said cuff (2).

12. Vital-sign detection device according to anyone of the preceding claims, wherein the sensor (13) includes different types of sensing components (15) for sensing a plurality of different vital signs.

13. Vital-sign detection device according to anyone of the preceding claims, wherein the sensor (13) includes at least one of the following sensing components (15): a sensing component for the detection of blood pressure, a sensing component for the detection of ECG, a sensing component for the detection of EMG, a sensing component for the detection of nerve impulses, a sensing component for the detection of bio-impedance, a sensing component for the detection of fluid flow and a sensing component for the detection of oxygen saturation.

14. Vital-sign detection device according to anyone of the preceding claims, wherein said sensor (13) includes at least one photodiode and at least one light source for measuring light transmission or reflection, wherein said photodiode and light source are accommodated in the interior chamber (8) of the bladder (3) and wherein the stretchable sheet material (5) is transparent for allowing the light source and photodiode visually detecting the body part through the stretchable sheet material (5).

15. Vital-sign detection device according to anyone of the preceding claims, wherein the sensor (13) includes at least one pressure sensor (4) measuring the pressure in the interior chamber (8) of the bladder (3) and/or the pressure of the bladder (3) against the body part.

16. Vital-sign detection device according to anyone of the preceding claims, wherein said cuff (2) and said bladder (3) are configured to snuggly fit onto and/or press-fit onto a finger of a human body.

17. Vital-sign detection device according to anyone of the preceding claims, wherein a data and/or sensor signal transmitter (22) is provided for transmitting detected signals and/or data derived therefrom to an external data processing and/or display unit (21).
